# EUROPEAN PATENT SPECIFICATION

(11) **EP 1 252 516 B1**
(45) Date of publication and mention of the grant of the patent: **14.04.2004**
(21) Application number: 01908442.5
(22) Date of filing: 02.02.2001
(51) Int. Cl.: G01N 33/53, B05D 7/24

(54) **METHOD OF TREATING A SURFACE OF AN OBJECT WITH A HYDROPHOBIN-CONTAINING SOLUTION**
VERFAHREN ZUR BEHANDLUNG EINER OBERFLÄCHE MIT EINER HYDROPHOBIN-ENTHALTENDEN LÖSUNG
PROCEDE POUR LE TRAITEMENT DE SURFACE D'UN OBJET AVEC UNE SOLUTION CONTENANT DE L'HYDROPHOBINE

(30) Priority: 04.02.2000 GB 0002663
(43) Date of publication of application: 30.10.2002
(73) Proprietor: Applied NanoSystems B.V., 9700 BC Groningen (NL); STICHTING VOOR DE TECHNISCHE WETENSCHAPPEN, 3527 JP Utrecht (NL)
(72) Inventor: DE VOCHT, Marcel, Leo, NL-3445 TE Woerden (NL); WÖSTEN, Herman, Abel, Bernard, NL-3705 SN Zeist (NL); WESSELS, Joseph, Gerard, Hubert, NL-9475 PG Midlaren (NL)
(74) Representative: Prins, Adrianus Willem
(86) International application number: PCT/NL2001/000084
(87) International publication number: WO 2001/057528

(56) References cited:
- WO-A-96/41882
- MARTIN G G ET AL: "Adsorption of a fungal hydrophobin onto surfaces as mediated by the associated polysaccharide schizophyllan" BIOPOLYMERS, vol. 49, 1999, pages 621-633, XP000999655
- DE VOCHT M.L.: "Structural characterization of the hydrophobin SC3, as a monomer and after self-asssembly at hydrophobic/hydrophilic interfaces" BIOPHYSICAL JOURNAL, vol. 74, April 1998 (1998-04), pages 2059-2068, XP000999824
- WESSELS: "Hydrophobins: proteins that change the nature of the fungal surface" ADV. MICROB. PHYSIOL., no. 38, 1997, pages 1-45, XP000999791 cited in the application
- WÖSTEN ET AL: "Hydrophobins, the fungal coat unravelled" BIOCHIMICA ET BIOPHYSICA ACTA , no. 1469, pages 79-86, XP000986270

## Description

The present invention relates to a method of treating a surface of an object with a hydrophobin-containing solution for providing the surface with a hydrophobin coating.

Hydrophobins are proteins known for their capability of forming a water-insoluble coating on a surface of an object. The adherence is so strong that the coating can not be removed by boiling in a 2% sodium dodecylsulfate (SDS) solution. Indeed, it has been suggested to coat a surface of, for example a biosensor, with a hydrophobin to modify the hydrophobic/hydrophillic nature of said surface.

Despite the alleged strong adherence of hydrophobin applicant has found that this is certainly not always the case, and that hydrophobin may be released from a coated surface under relatively mild conditions, in particular those which may also occur during the intended and normal use of the object.

The object of the present invention is to provide a method according to the preamble which yields a surface coated with hydrophobins which remain firmly bound to said surface under a wider range of conditions.

To this end, the method according to the present invention is characterized in that the object is chosen from the group consisting of a window, a contact lens, a biosensor, a medical device, a container for performing an assay or storage, the hull of a vessel or a frame or bodywork of a car, and a solid particle whereby the surface of said object after being coated with hydrophobin is treated at a temperature of at least 30°C, which temperature does not exceed 80°C.

Surprisingly it has been found that a thermal treatment reduces the likelyhood that the hydrophobin is released from the surface of the object. Without being bound to any theory, applicant is of the opinion that, because this change appears to be permanent, this behaviour involves a conformational change.

Martin, G.G. et al. (Biopolymers 49, pp. 621-633 (1999)) describe the analysis of secreted fungal components (hydrophobin and schizophyllan) on solid surfaces, in particular Parafilm® and mica. The mica sheets are treated with 2% SDS at 90°C. It is concluded that the water contact angle data indicate that schizophyllan and hydrophobin form a SDS-resistant coating on mica. No mention is made that untreated hydrophobin can be eluted under mild conditions.

De Vocht, M.L. et al. (Biophysical Journal, 74, pp. 2059-2068 (1998)) similarly describe treating a Teflon surface coated with hydrophobin SC3 with 2% SDS at 100°C.

In the present application the term "window" is meant to be a framed plastic or glass window, such as a windshield of a vehicle, a window of a building, or a spectacle lens. A container for storage is, for example a container, such as a bottle, for a substance of biological origin. The term also encompasses microtiter plates for performing assays, such as immunoassays. The term "medical device" is defined as a device which is to be contacted with a tissue or bodily fluid of a (live) animal, such as a catheter or a surgical device such as a trocar, an endoscope, a clip, cutting tool or suture wire. The solid particle may be a paint particle or a particle used for analytical purposes, such as a spherical gold or latex particle, these particles well known in the art of assays and in particular immunoassays.

Hydrophobins are a well-defined class of proteins (ref. 1) capable of self-assembly at a hydrophobic-hydrophilic interface, and having a conserved sequence

Xₙ-C-X₅₋₉-C-C-X₁₁₋₃₉-C-X₈₋₂₃-C-X₅₋₉-C-C-X₆₋₁₈-C-Xₘ

X, of course, represents any amino acid, and n and m, of course, independently represent an integer. In general, a hydrophobin has a length of up to 125 amino acids. The cysteine residues (C) in the conserved sequence are part of disulfide bridges. In the present invention, the term hydrophobin has a wider meaning to include functionally equivalent proteins, and encompasses a group of proteins comprising the sequence or parts thereof

Xₙ-C-X₁₋₅₀-C-X₀₋₅-C-X₁₋₁₀₀-C-X₁₋₁₀₀-C-X₁₋₅₀-C-X₀₋₅-C-X₁₋₅₀-C-Xₘ

still displaying the characteristic of self-assembly at a hydrophobic-hydrophilic interface resulting in a protein film. In accordance with the definition of the present invention, self-assembly can be detected by adsorbing the protein to Teflon and use Circular Dichroism to establish the presence of a secundary structure (in general α-helix) (ref. 2). The formation of a film can easily be established by incubating a Teflon sheet in the protein solution followed by at least three washes with water or buffer (ref. 3). The protein film can be visualised by any method, such as labeling with a fluorescent compound or by the use of fluorescent antibodies, as is well established in the art. m and n may have values ranging from 0 to 2000. Included in the definition are fusion-proteins of a hydrophobin and another protein.

The present invention is particularly suitable in those instances where the object is or may be in contact with a surfactant, such as a windshield or a container.

Without wishing to be bound to any particular theory, the applicant is of the opinion that the change in secondary structure is a change from an alpha-helix state to a beta-sheat state, as can be determined using spectroscopic techniques such as circular dichroism (ref. 2). To determine a suitable temperature for inducing the irreversible change, the person skilled in the art can rely on easy to perform routine experiments. A surface is coated with a desired hydrophobin, the surface is treated at a certain temperature for any length of time, such as 10 minutes. After that, the surface is rinsed at ambient temperature with a solution containing 0,1% Tween 20. After rinsing the presence of hydrophobin is detected using any suitable method. Suitable methods are for example the use of (labeled) antibodies against hydrophobin. Alternatively, the hydrophobin used to coat the surface is a fluorescently or radioactively labeled hydrophobin.

Preferably, the treatment is performed at a temperature of at least 30°C in the presence of a surfactant.

While a surfactant may elute hydrophobins at ambient temperature, it appears to effect a change in secondary structure at an elevated temperature, such as at least 35°C, rendering the hydrophobin insoluble, even in the presence of a surfactant. This change is permanent, that is, even after the coated surface is returned to ambient temperature. The treatment may be carried out in the presence of hydrophobin in solution.

Generally the surfactant is present in a concentration of at least 0.001% wt./vol., preferably at least 0.01% wt./vol., more preferably 0.1% wt./vol. and with the highest preference at least 1% wt./vol.

At higher concentrations the change in secondary structure occurs more rapidly.

For objects made of thermally sensitive material, such as thermoplastics, the temperature preferably does not exceed 65°C.

This saves both energy and, where applicable, prevents deformation of the shape of the object to be coated. Lower temperatures may require treatment for a longer time. In addition, it is completely within the capabilities of a person skilled in the art to select a hydrophobin which meets the required standard regarding non-specific binding. This embodiment allows for the coating of containers, in particular microtiter plates, as used in assays, such as ELISAs, which containers are often made out of a thermoplastic material with a relatively low melting temperature. It is remarked that in various assays, such as ELISAs, and for various objects use is made of a detergent and it would not be possible to employ a hydrophobin without the method according to the present invention.

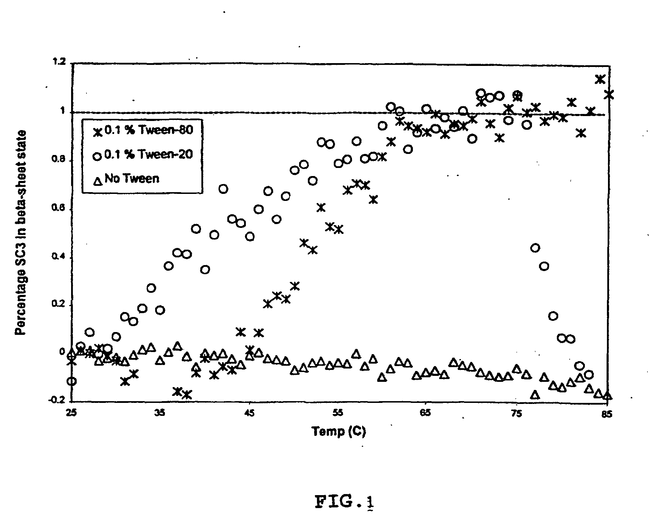

The present invention will now be illustrated using the following examples and with reference to the drawing where
fig. 1 shows the effect of Tween and temperature on the induction of the stable beta-sheet form of SC3 at a Teflon surface; and
fig. 2 depicts the amount of SC3 remaining bound to a Teflon surface.

### METHODS

### A) Purification of hydrophobin SC3

The hydrophobin SC3 was purified from the culture medium of strain 4-40 of *Schizophyllum commune* (CBS 340.81) as described (1, 4). Before use, the freeze-dried SC3 was disassembled with pure TFA and dried in a stream of nitrogen. The monomeric protein was then dissolved in the buffer as specified under B) or in water.

### B) Secondary structure measurements

The secondary structure of the SC3 was studied with circular dichroism spectroscopy (CD). The CD-spectra were recorded over the wavelength region 190-250 nm on an Aviv 62A DS CD spectrometer (Aviv Associates, Lakewood, New Jersey, USA), using a 1-mm quartz cuvette. The sample compartment was continuously flushed with N₂ gas and the temperature was kept varied. 10 scans were averaged, using a bandwidth of 1 nm, a stepwidth of 1 nm, and 1 sec averaging per point. The spectra were corrected using a reference solution without the protein. Typically a protein concentration of 10 µM in 50 mM phosphate pH 7.0 was used. For spectra of SC3 bound to a hydrophobic support, 130 nm non-stabilized colloidal Teflon spheres (Dupont de Nemours, Geneva, Switzerland) in water were added to the solution, following a known procedure (2).

### C) Binding to Teflon

The coating of Teflon by SC3 was assessed essentially as described by Wösten et al. (3). Thoroughly cleaned (ref. 3) Teflon sheets (Norton Fluorplast B.V., Raamsdonksveer, The Netherlands) were incubated for 16 hours in 20 µg/ml ³⁵S-labelled SC3 in water, followed by three washes with water for 10 minutes each. The amounts of ³⁵S-labelled protein were determined by scintillation counting.

### EXAMPLE 1

50 µg/ml SC3 in 50 mM phosphate buffer (pH = 7.0) was mixed with 130 nm unstabilized colloidal Teflon spheres (Dupont de Nemours, Geneva, Switzerland) at 25°C. SC3 adsorbed to the surface of the Teflon and attained the α-helical state (calculated surface coverage 9%).

Samples of Teflon spheres coated with SC3 were then gradually heated to 85°C (1°C/min) in the presence or absence of a detergent and the CD-signal was followed. The CD-signal was normalised and plotted against the temperature (Fig.).

It was observed that SC3 remained in the α-helical state in the absence of detergent. However, in the presence of 0.1% Tween-80 50% of the SC3 changed from the monomeric state to the assembled β-sheet state at ± 53°C. Complete transition was obtained at about 70°C. A similar effect was observed in the presence of 0.1% Tween-20. However, 50% of SC3 changed its structure at ± 39°C, while complete transition was observed at 63°C.

After heating the samples to 85°C, the samples were cooled to 25°C. In contrast to samples that had not been heated (see above), SC3 did not desorb but rather remained attached in the β-sheet conformation. In the absence of detergent SC3 remained attached in the α-helical state. It is noted that the drop above 75°C for 0.1% Tween-20 was an artefact caused by settling of the spheres.

From the experiment it can be concluded that, under the above experimental conditions, Tween-20 and Tween-80 both trigger the conformational change to β-sheet and do so at different temperatures (at 63°C in 0.1% Tween-20 or at 70°C in 0.1% Tween-80).

Surprisingly, it has been found that this conformational change is needed to obtain strong binding to hydrophobic surfaces.

### EXAMPLE 2

Teflon sheets (2 cm², thickness 0.25 mm) were incubated in 20 µg/ml ³⁵S-labelled SC3 overnight at room temperature. The SC3-coated sheets were subsequently washed with water at room temperature. The sheets were then treated with 2% Tween 20 (pH 7.0) or water (control), either at room temperature or 100°C (control) for 30 min. The amount of radioactive SC3 released from the Teflon sheet was determined. Percentages are relative to the amount of radioactivity oriainallv bound to the sheet.

| | % SC3 released | |
|---|---|---|
| | room temperature | 100°C |
| 2% Tween 20 | 78% | 6% |
| Water (control) | 6% | 7% |

When the sheets (treated at room temperature or 100°C in the absence or presence of Tween 20) were subsequently incubated at room temperature for 30 min. with the respective wash solution, no additional SC3 desorbed from the surface. From this experiment it can be concluded that after a treatment with heat and surfactant, adsorbed hydrophobin can no longer be eluted with surfactant and will be more suitable as a coating for the above objects.

### EXAMPLE 3

350 ul containing 35 ug SC3 and 0.23 m² colloidal Teflon were incubated in a cuvette at a constant temperature, as indicated in the table below. The Circular Dichroism-spectrum was determined between 190 nm and 250 nm. This revealed all of the temperatures indicated in the table a typical α-helical spectrum. Then surfactant was added to the concentration indicated in the table. The CD-spectrum was followed in time and the respective times to reach the β-sheet state are indicated in the table.

| Detergent | Concentration | Temperature | Transition to β-sheet |
|---|---|---|---|
| SDS | 2% | 85°C | 15' |
| SDS | 2% | 65°C | ≈30' |
| SDS | 2% | 45°C | 40' |
| SDS | 2% | 25°C | >24 hour |
| Tween-80 | 0.1% | 85°C | >5' |
| Tween-80 | 0.1% | 65°C | 45' |
| Tween-80 | 0.1% | 45°C | 120' |
| Tween-80 | 0.1% | 25°C | >24 hour |
| Tween-80 | 0.01% | 85°C | ≈5 hours |
| Tween-80 | 0.2% | 85°C | <5' |
| Tween-80 | 0.5% | 65°C | ≈40' |
| Tween-20 | 0.1% | 85°C | ≈15' |
| Tween-20 | 0.1% | 65°C | ≈25' |
| Tween-20 | 0.1% | 45°C | ≈250' |
| Tween-20 | 0.1% | 25°C | > 7 hours |

Conclusions:
- At higher concentration Tween-80 the rate increases.
- At higher temperatures the rate increases.

### EXAMPLE 4

Teflon sheets (2 cm², thickness 0.25 mm) were incubated in 10 µg/ml labelled (³⁵S) SC3 in water at room temperature (RT), followed by ample washing with water. The sheets were subsequently incubated for 30 minutes in water at the temperature indicated.

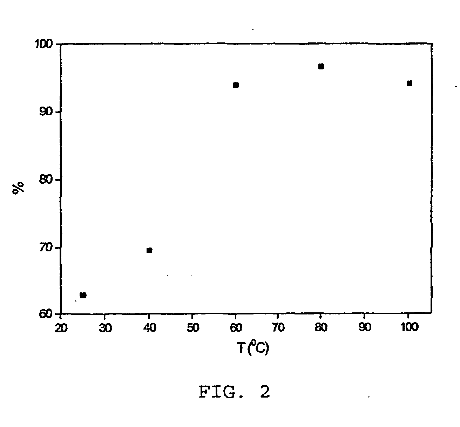

To determine the percentage of SC3 remaining firmly bound to the Teflon sheets, half of them were extracted for 30 minutes with 0.1% Tween-20 in water while the other half was used as the respective control. The percentage of SC3 remaining (with respect to the respective control) is plotted in fig. 2. From this figure it can be concluded that incubation at a temperature of over 30°C increases the strength of binding to the surface. It can also be seen that a temperature of about 60°C for 30 minutes suffices for excellent binding.

### REFERENCES

1. Wessels, J.G.H. (1997) in Adv. Microb. Physiol. 38, 1-45.
2. De Vocht, M.L., et al. (1998) in Biophys. J. 74, 2059-68.
3. Wösten, H.A.B., et al. (1994) in Embo. J. 13, 5848-54.
4. Wösten, H.A.B., et al. (1993) in Plant Cell 5, 1567-74.

## Claims

1. Method of treating a surface of an object with a hydrophobin-containing solution for providing the surface with a hydrophobin coating, **characterized in that** the object is chosen from the group consisting of a window, a contact lens, a biosensor, a medical device, a container for performing an assay or storage, the hull of a vessel or a frame or bodywork of a car, and a solid particle whereby the surface of said object after being coated with hydrophobin is treated at a temperature of at least 30°C, which temperature does not exceed 80°C.

2. Method according to claim 1, **characterized in that** the treatment at a temperature of at least 30°C is performed in the presence of a surfactant.

3. Method according to claim 1 or 2, **characterized in that** the surfactant is present in a concentration of at least 0.001% wt./vol., preferably at least 0.01% wt./vol., more preferably 0.1% wt./vol. and with the highest preference at least 1% wt./vol.

4. Method according to any of the preceding claims, **characterized in that** the temperature does not exceed 65°C.

## Patentansprüche

1. Verfahren zum Behandeln einer Oberfläche eines Gegenstands mit einer Hydrophobin enthaltenden Lösung zum Versehen der Oberfläche mit einer Hydrophobinbeschichtung, **dadurch gekennzeichnet, dass** der Gegenstand aus der Gruppe gewählt wird, die ein Fenster, eine Kontaktlinse, einen Biosensor, eine medizinische Vorrichtung, einen Behälter zur Durchführung eines Versuchs oder einer Lagerung, den Rumpf eines Schiffes oder einen Rahmen oder eine Karosserie eines Personenkraftwagens, und ein festes Teilchen umfasst, wobei die Oberfläche des Gegenstands nach dem Beschichten mit Hydrophobin bei einer Temperatur von mindestens 30 °C behandelt wird, wobei die Temperatur 80 °C nicht übersteigt.

2. Verfahren nach Anspruch 1, **dadurch gekennzeichnet, dass** die Behandlung bei einer Temperatur von mindestens 30 °C in Anwesenheit eines oberflächenaktiven Stoffes durchgeführt wird.

3. Verfahren nach Anspruch 1 oder 2, **dadurch gekennzeichnet, dass** der oberflächenaktive Stoff in einer Konzentration von mindestens 0,001 % Gewicht zu Volumen, vorzugsweise mindestens 0,01 % Gewicht zu Volumen, besser 0,1 % Gewicht zu Volumen und am besten mindestens 1 % Gewicht zu Volumen anwesend ist.

4. Verfahren nach einem der vorhergehenden Ansprüche, **dadurch gekennzeichnet, dass** die Temperatur 65 °C nicht übersteigt.

## Revendications

1. Un procédé de traitement de la surface d'un objet avec une solution contenant de l'hydrophobine afin de doter la surface d'un revêtement d'hydrophobine, **caractérisé en ce que** l'objet est choisi parmi le groupe comprenant une fenêtre, une lentille de contact, un biocapteur, un dispositif médical, un récipient pour effectuer un dosage ou un stockage, la coque d'un navire ou un châssis ou une carrosserie de voiture, et une particule solide, par lequel la surface dudit objet, après avoir été revêtue de l'hydrophobine, est traitée à une température d'au moins 30 °C, laquelle température ne dépasse pas 80 °C.

2. Un procédé selon la revendication 1, **caractérisé en ce que** le traitement à une température d'au. moins 30 °C est réalisé en présence d'un agent tensioactif.

3. Un procédé selon la revendication 1 où 2, **caractérisé en ce que** l'agent tensioactif est présent à une concentration d'au moins 0,001 % en poids/volume, de préférence d'au moins 0,01 % en poids/volume, plus préférentiellement de 0,1 % en poids/volume et idéalement d'au moins 1 % en poids/volume.

4. Un procédé selon l'une quelconque des revendications précédentes, **caractérisé en ce que** la température ne dépasse pas 65 °C.
